# EUROPEAN PATENT APPLICATION

(11) **EP 4 197 491 A1**
(43) Date of publication of application: **21.06.2023**
(21) Application number: 21216070.9
(22) Date of filing: 20.12.2021
(51) Int. Cl.: A61F 2/02

(54) **MODULAR IMPLANTABLE DEVICE FOR THE MACROENCAPSULATION OF CELLS**

(71) Applicant: Technische Universität Dresden, 01069 Dresden (DE); Leibniz-Institut für Polymerforschung Dresden e.V., 01069 Dresden (DE)
(72) Inventor: Ludwig, Barbara, 01326 Dresden (DE); Bornstein, Stefan, 01309 Dresden (DE); Werner, Carsten, 01324 Dresden (DE); Heller, Carolin, 01099 Dresden (DE); Welzel, Petra, 01109 Dresden (DE)
(74) Representative: Ulrich, Thomas Franz

(57) **Abstract**

A modular implantable device for the macroencapsulation of cells, comprising one bracket, one cell module one oxygen module, one immuno-isolating membrane.

The cell module comprises a hydrogel or a solid carrier comprising a cell arrangement pattern, in particular a microwell array.

## Description

The present invention pertains to a modular implantable device for the macroencapsulation of cells and a method for preparing such device.

Diseases and disorders related to endocrine function are often associated with the destruction of endogeneous endocrine cells, in particular caused by an autoimmune reaction or genetic mutations. The progressive loss of endocrine function can result in a major problem for human health and significantly threaten life quality. Present therapies are primarily based on supplying the body with allogeneic, xenogeneic or recombinantly produced hormones to at least partially restore the impaired or lost biological activity and to ameliorate the constraints associated therewith.

Diabetes mellitus is a metabolic disease affecting millions of people worldwide. The world health organisation (WHO) and the International Diabetes Federation estimated in 2019 about 463 million diabetic patients worldwide corresponding to 9.3 % in the adult population. The incidence of diabetes is constantly increasing worldwide.

The autoimmune disease diabetes mellitus type 1 is characterized by a T-cell mediated immune reaction resulting in a successive destruction of the beta cells of the pancreas. As a consequence, the endogenous production of insulin is insufficient to regulate the blood glucose level. The deficiency of endogenous insulin production leads to hyperglycaemia and a tendency to ketoacidosis. Before insulin was first isolated in 1921, the diagnosis of type 1 diabetes limited the life expectancy to only a few months. In the century since its discovery, developments in the isolation of insulin and delivery as well as advantages in glycaemic control technologies have significantly altered and improved the treatment of diabetes mellitus type 1. Currently, whole pancreas and islet transplantation are the only clinically available means of beta cell replacement. While pancreas transplantation is mainly performed simultaneously with a kidney transplantation in patients with diabetes mellitus type 1 and end-stage renal disease, the intraportal transplantation of isolated pancreatic islets is an established therapy for treatment of diabetes mellitus type 1 patients with highly unstable metabolic control. Despite a steady improvement of graft survival and function in recent years, there are several factors hampering a more widespread application of islet transplantation. The three most important and yet unresolved problems are insufficient oxygen supply of the isolated and transplanted islets due to a lack of revascularization in the early post-transplantation phase, permanent exposure to harmful allo- and autoimmunological and inflammatory processes, as well as a lack of a sufficient number of donor organs for islet isolation.

A major issue with the transplantation of endocrine cells is the requirement to modulate the recipient's immune system in order to avoid or diminish the immunological and inflammatory response to donor cells. It is therefore desirable to as much as possible eliminate exposure of transplanted tissue or cells to the recipient's immune system. One promising way is the encapsulation of the cells or tissue prior to transplantation. This strategy creates an immunologically shielded space preventing the passage of the encapsulated components while an immuno-isolating barrier at the same time allows donor and recipient tissue to exchange oxygen, nutrients and hormones. Furthermore, the encapsulation approach circumvents the shortage of donor organs since alternative cell sources can be used for transplantation, such as xenogeneic or stem cell-derived hormone producing cells or tissues. However, a problem arising from the encapsulation of cells is the need to ensure sufficient oxygen supply of the encapsulated cells from the moment of implantation until the surrounding environment is revascularized to provide oxygen by diffusion from host capillaries in close proximity to the encapsulated cells. It usually takes several days to weeks until normoxic conditions are established by revascularization of the tissue surrounding an implant. An acute oxygen deficiency occurring in the early post transplantation phase cannot be reversed by the revascularization of the tissue.

To overcome these obstacles, the present invention provides a modular implantable device for the macroencapsulation of cells which can be used as a bioartificial organ comprising cells of interest, in particular endocrine cells of interest.

The present invention pertains to a modular implantable device for the macroencapsulation of cells comprising: at least one bracket configured for accommodating at least one cell module within the device, at least one oxygen module, and at least one immuno-isolating membrane, wherein the at least one oxygen module is contained in the device in a shell formed by the at least one bracket and the at least one immuno-isolating membrane, optionally together with the at least one cell module.

The modular implantable device for the macroencapsulation of cells according to the present invention advantageously allows to provide a recipient subject with cells which are accommodated within an immunologically shielded space formed by the at least one bracket and the at least one immuno-isolating membrane. The at least one immuno-isolating membrane allows passage of gases, such as carbon dioxide and oxygen, nutrients and hormones but hinders the migration of cells and macromolecules into and out of the device. In particular, the at least one immuno-isolating membrane prevents cells and macromolecules of the immune system to come into contact with cells within the modular implantable device of the present invention. The additional presence of the at least one oxygen module within the immunologically shielded space of the device allows to supply the cells with sufficient oxygen already directly after transplantation of the device, and thus in a phase in which the recipient's tissue surrounding the implanted device is not sufficiently vascularized to provide enough oxygen by diffusion through the at least one immuno-isolating membrane. The at least one oxygen module in the device advantageously bridges the lack of oxygen supply in the early post translational phase, that means from the moment of implantation until the tissue surrounding the implant is sufficiently revascularized to supply the cells within the device via free diffusion of oxygen. In particularly preferred embodiments of the present invention, the at least one immuno-isolating membrane can further comprise bioactive compounds, in particular growth factors, preferably at least one pro-angiogenic/vascularising growth factor and/or at least one trophic growth factor, in order to expedite vascularization of the modular implantable device.

According to a preferred embodiment of the present invention, the modular implantable device comprises at least one cell module. Particularly preferred, the at least one cell module is arranged in the at least one bracket configured for accommodating at least one cell module in the device.

Preferably, the modular implantable device comprises at least two cell modules, in particular comprises two cell modules. In a further preferred embodiment of the present invention, the modular implantable device comprises two cell modules which are arranged in the at least one bracket configured for accommodating at least one cell module in the device. Preferably, the two cell modules are arranged in two brackets configured for accommodating at least one cell module in the device.

In a particularly preferred embodiment of the present invention, the at least one cell module comprises a hydrogel or solid carrier.

According to the present invention, the cell module, in particular the hydrogel or solid carrier, serves to harbour cells or cell clusters within the modular implantable device.

In preferred embodiment of the present invention, the at least one bracket configured for accommodating at least one cell module in the device is part of a scaffold module.

In another preferred embodiment of the present invention, the at least one bracket configured for accommodating at least one cell module in the device is part of a membrane module comprising at least one immuno-isolating membrane. According to this particular embodiment, the at least one bracket is configured to support the at least one immuno-isolating membrane and further to accommodate at least one cell module within the device.

The present invention also encompasses embodiments in which at least one bracket configured for accommodating at least one cell module in the device is part of a scaffold module and at least one bracket configured for accommodating at least one cell module in the device is part of a membrane module comprising at least one immuno-modulating membrane.

In a further preferred embodiment of the present invention, the cell module, preferably the hydrogel or solid carrier, comprises a cell arrangement pattern.

Preferably, the cell arrangement pattern is an array of cells or cell clusters, in particular a one-, two-, or three-dimensional array of cells or cell clusters, such as in the form of a row (one-dimensional array), a grid (two-dimensional array) or a stacked array (three-dimensional array). Particularly preferred, the cells or cell clusters are embedded in or enclosed by a hydrogel to form the cell arrangement pattern.

In a preferred embodiment, the cells or cell clusters are positioned on or inside elevations of the hydrogel or solid carrier to form the cell arrangement pattern.

In another preferred embodiment of the present invention, the cells or cells clusters are positioned in indentations of the hydrogel or solid carrier to form the cell arrangement pattern. Preferably, the indentations of the hydrogel or solid carrier are wells, in particular microwells. Particularly preferred, the cells or cell clusters are positioned in a microwell array, such as a periodic regular microwell array, preferably in a microwell hydrogel array, to form the cell arrangement pattern.

The positioning of the cells or cell clusters within the cell module, preferably within the hydrogel or solid carrier, in particular in the cell arrangement pattern, primarily serves to ensure that all individual cells or cell clusters within the cell module are provided with sufficient nutrients and oxygen by free diffusion. In case the cells or cell clusters are positioned too close with respect to one another, the nutrient and oxygen availability by free diffusion is hampered by the neighbouring cells or cell clusters. Thus, in a particularly preferred embodiment, the cells or cell clusters are positioned in the cell module, preferably in the hydrogel or solid carrier, in particular in the cell arrangement pattern, preferably in the microwell array, preferably in the microwell hydrogel array, with respect to each other in a compromise between sufficient nutrient and oxygen supply on the one side and a maximum cell number per area on the other side.

The specific positioning of the cells or cell clusters within the cell module, preferably within the hydrogel or solid carrier, in particular in the cell arrangement pattern, advantageously allows an efficient oxygen supply to the cells, in particular a supply with oxygen provided by the at least one oxygen module. The specific positioning of the cells or cell clusters within the cell module, preferably within the hydrogel or solid carrier, in particular in the cell arrangement pattern, further ensures efficient supply of oxygen from the vasculature surrounding the modular implantable device by diffusion via the at least one immuno-isolating membrane once the oxygen reservoir in the at least one oxygen module is depleted.

In a preferred embodiment of the present invention, the hydrogel comprises, in particular consists of, a natural, semi-synthetic or synthetic polymer. Preferably, the hydrogel comprises, in particular consists of, a natural, semi-synthetic or synthetic polymer selected from the group consisting of glycosaminoglycans (GAGs), agarose, chitosan, alginate, collagen, poly(amino acid)s, poly(peptoid)s and derivatives thereof, poly(ethylene glycol)s (PEG), poly(oxazoline)s (POX), synthetic sulfonated or sulfated polymers, e.g. sulfonated or sulfated poly(acrylic acid) or poly(styrene sulfonic acid), acrylates, poly(N-vinyl-2-pyrrolidinone) (PNVP), poly(acrylamide) (PAM), poly(vinyl alcohol) (PVA), poly(ethylene oxide) (PEO), poly(N-isopropyl acrylamide) (PNIPAM), poly(methacrylic acid) (PMAA), and derivatives thereof, and combinations thereof.

According to a preferred embodiment of the present invention, the hydrogel or solid carrier further comprises at least one bioactive compound. Preferably, the at least one bioactive compound is a compound effecting survival, functionality and/or maturation of cells.

Preferably, the hydrogel or solid carrier comprises at least one hydrogen peroxide decomposition catalyst, preferably a hydrogen peroxide decomposing enzyme, in particular catalase.

In a preferred embodiment of the present invention, the cell arrangement pattern, preferably the microwell array and/or the microwell has a polygonal geometry. In a preferred embodiment of the present invention, the cell arrangement pattern, preferably the microwell array and/or the microwell has an equilateral triangle, round, square or hexagonal geometry. According to a particularly preferred embodiment of the present invention, the cell arrangement pattern, preferably the microwell array and/or the microwell has a hexagonal geometry.

According to a preferred embodiment of the present invention, the cells or cell cluster are arranged in the cell module, preferably in the hydrogel or solid carrier, in particular in the cell arrangement pattern, in a pattern having a polygonal geometry. Preferably, the cells or cell clusters are arranged in the cell module, preferably in the hydrogel or solid carrier, in particular in the cell arrangement pattern, in a pattern having an equilateral triangle, round, square or hexagonal geometry. Particularly preferred, the cells or cell clusters are arranged in the cell module, preferably in the hydrogel or solid carrier, in particular in the cell arrangement pattern, in a pattern having a hexagonal geometry.

In a preferred embodiment of the present invention, the cell module, preferably the hydrogel or solid carrier, in particular the cell arrangement pattern, comprises a plurality of patterns having a polygonal geometry. Preferably, the cell module, preferably the hydrogel or solid carrier, in particular the cell arrangement pattern, comprises a plurality of patterns having a hexagonal geometry. In a particularly preferred embodiment, the individual hexagonal patterns are arranged in the cell module, preferably in the hydrogel or solid carrier, in particular in the cell arrangement pattern, in a honeycomb pattern.

In a further preferred embodiment of the present invention, the cell module, preferably the hydrogel or solid carrier, in particular the cell arrangement pattern, comprises at least one, preferably at least two, preferably at least three, preferably at least four, preferably at least five, preferably at least 10, preferably at least 15, preferably at least 20, preferably at least 25, preferably at least 50, patterns having a hexagonal geometry. In particularly preferred embodiment, the cell module comprises one, preferably three, preferably seven, preferably 19, preferably 37, preferably 61, patterns having a hexagonal geometry.

Preferably, the patterns having a hexagonal geometry are arranged with respect to each other to form a hexagonal superstructure, in particular a honeycomb superstructure, in the cell module, preferably in the hydrogel or solid carrier, in particular in the cell arrangement pattern. Particularly preferred, the cell arrangement pattern is a hexagonal superstructure, in particular honeycomb superstructure, formed by a plurality of patterns having a hexagonal geometry. Preferably, the cell arrangement pattern is a hexagonal superstructure, in particular honeycomb superstructure, formed by at least one, preferably at least two, preferably at least three, preferably at least four, preferably at least five, preferably at least 10, preferably at least 15, preferably at least 20, preferably at least 25, preferably at least 50, patterns having a hexagonal geometry. In another preferred embodiment, the cell arrangement pattern is a hexagonal superstructure, in particular honeycomb superstructure, formed by one, preferably three, preferably seven, preferably 19, preferably 37, preferably 61, patterns having a hexagonal geometry.

The use of a cell arrangement pattern, preferably a microwell array and/or a microwell, with a hexagonal geometry allows a high packing density of cells within the cell module while allowing sufficient nutrient and oxygen supply via diffusion.

In a preferred embodiment of the present invention, the wells of the microwell array, in particular the microwell hydrogel array, have a width of at most 350 µm, preferably at most 300 µm, preferably at most 200 µm, preferably at most 190 µm, preferably at most 180 µm, preferably at most 170 µm, preferably at most 160 µm, preferably at most 150 µm, preferably at most 140 µm, preferably at most 130 µm, preferably at most 120 µm, preferably at most 110 µm, preferably at most 100 µm, preferably at most 90 µm, preferably at most 80 µm, preferably at most 70 µm, preferably at most 60 µm, preferably at most 50 µm.

In a further preferred embodiment of the present invention, the wells of the microwell array, in particular the microwell hydrogel array are spaced from each other by at least 5 µm, preferably at least 10 µm, preferably at least 15 µm, preferably at least 20 µm, preferably at least 25 µm, preferably at least 30 µm, preferably at least 35 µm, preferably at least 40 µm, preferably at least 45 µm, preferably at least 50 µm, preferably at least 75 µm, preferably at least 100 µm.

Particularly preferred, the wells of the microwell array, in particular the microwell hydrogel array, have a width of at most 350 µm, preferably at most 300 µm, preferably at most 200 µm, preferably at most 150 µm, preferably at most 125 µm, preferably at most 100 µm, and/or are spaced from each other by at least 5 µm, preferably at least 10 µm, preferably at least 25 µm.

According to a preferred embodiment of the present invention, the wells of the microwell array, in particular the wells of the microwell hydrogel array, have a polygonal geometry. Preferably, the wells of the microwell array, in particular the wells of the microwell hydrogel array, have an equilateral triangle, round, square or hexagonal geometry. Particularly preferred, the wells of the microwell array, in particular the wells of the microwell hydrogel array, have a hexagonal geometry.

In a particularly preferred embodiment of the present invention, the wells of the microwell array, in particular the wells of the microwell hydrogel array, have a hexagonal geometry and are arranged with respect to each other to form at least one hexagonal unit comprising a plurality of wells having a hexagonal geometry. Preferably, the wells of the microwell array, in particular the wells of the microwell hydrogel array, have a hexagonal geometry and are arranged with respect to each other in a honeycomb pattern.

In a further preferred embodiment of the present invention, the cell module comprises at least one, preferably at least two, preferably at least three, preferably at least four, preferably at least five, preferably at least 10, preferably at least 15, preferably at least 20, preferably at least 25, preferably at least 50, hexagonal units comprising a plurality of wells having a hexagonal geometry. In particularly preferred embodiment, the cell module comprises one, preferably three, preferably seven, preferably 19, preferably 37, preferably 61, hexagonal units comprising a plurality of wells having a hexagonal geometry. Preferably, the hexagonal units comprising a plurality of wells having a hexagonal geometry of the cell module, particular of the hydrogel or solid carrier, in particular of the cell arrangement pattern, are arranged with respect to each other to form a hexagonal superstructure, in particular a honeycomb superstructure.

The arrangement of cells or cell clusters in such hexagonal superstructure of hexagonal units comprising a plurality of wells having a hexagonal geometry advantageously allows for a high surface coverage of the cell module and a high cell density.

In a preferred embodiment, the wells of the microwell array, in particular the microwell hydrogel array, have a flat bottom. In another preferred embodiment of the present invention, the wells of the microwell array, in particular the microwell hydrogel array, have a concave bottom.

In a further preferred embodiment of the present invention, the surface of the cells or surface of the cell clusters in the cell module, in particular in the hydrogel or solid carrier, in particular in the cell arrangement pattern, preferably in the microwell array and/or in the microwell, is spaced from the at least one immuno-isolating membrane by at most 500 µm, preferably at most 450 µm, preferably at most 400 µm, preferably at most 350 µm, preferably at most 300 µm, preferably at most 250 µm, preferably at most 225 µm, preferably at most 200 µm, preferably at most 175 µm, preferably at most 150 µm, preferably at most 125 µm, preferably at most 100 µm, preferably at most 90 µm, preferably at most 80 µm, preferably at most 70 µm, preferably at most 60 µm, preferably at most 50 µm, preferably at most 40 µm, preferably at most 30 µm, preferably at most 20 µm, preferably at most 10 µm. Particularly preferred, the cells or the cell clusters are in direct contact with the at least one immuno-isolating membrane.

According to a preferred embodiment of the present invention, the surface of the cells or the surface of the cell clusters in the cell module, in particular in the hydrogel or solid carrier, in particular in the cell arrangement pattern, preferably in the microwell array and/or in the microwell, is spaced from the at least one immuno-isolating membrane by at least 10 µm, preferably at least 20 µm, preferably at least 30 µm, preferably at least 40 µm, preferably at least 50 µm, preferably at least 60 µm, preferably at least 80 µm, preferably at least 90 µm, preferably at least 100 µm, preferably at least 125 µm, preferably at least 150 µm, preferably at least 175 µm, preferably at least 200 µm, preferably at least 250 µm.

In a preferred embodiment of the present invention, the surface of the cells or the surface of the cell clusters in the cell module, in particular in the hydrogel or solid carrier, in particular in the cell arrangement pattern, preferably in the microwell array and/or in the microwell, is spaced from the at least one oxygen module by at most 500 µm, preferably at most 450 µm, preferably at most 400 µm, preferably at most 350 µm, preferably at most 300 µm, preferably at most 250 µm, preferably at most 225 µm, preferably at most 200 µm, preferably at most 175 µm, preferably at most 150 µm, preferably at most 125 µm, preferably at most 100 µm, preferably at most 90 µm, preferably at most 80 µm, preferably at most 70 µm, preferably at most 60 µm, preferably at most 50 µm.

According to a further preferred embodiment of the present invention, the surface of the cells or the surface of the cell clusters in the cell module, in particular in the hydrogel or solid carrier, in particular in the cell arrangement pattern, preferably in the microwell array and/or in the microwell is spaced from the at least one oxygen module by at least 50 µm, preferably at least 60 µm, preferably at least 80 µm, preferably at least 90 µm, preferably at least 100 µm, preferably at least 125 µm, preferably at least 150 µm, preferably at least 175 µm, preferably at least 200 µm, preferably at least 250 µm.

In a preferred embodiment of the present invention, the wells of the microwell array, in particular the wells of the microwell hydrogel array or the solid carrier, are coated. Preferably, the wells of the microwell array, in particular the wells of the microwell hydrogel array or the solid carrier, are coated with at least one component of the extracellular matrix (ECM). In a particularly preferred embodiment, the wells of the microwell array, in particular the wells of the microwell hydrogel array or solid carrier, are coated with at least one component of the extracellular matrix (ECM) selected from the group consisting of collagen, elastin, laminin, fibronectin, heparan sulfate, chondroitin sulfate, keratan sulfate and hyaluronic acid. In a further preferred embodiment, the wells of the microwell array, in particular the wells of the microwell hydrogel array or solid carrier, are coated with at least one ECM-mimicking peptide sequence. In a particularly preferred embodiment, the wells of the microwell array, in particular the wells of the microwell hydrogel array or the solid carrier, are composed of at least one component of the extracellular matrix (ECM).

According to a particularly preferred embodiment of the present invention, the cells or cell clusters in the cell module, in particular in the hydrogel or solid carrier, in particular in the cell arrangement pattern, preferably in the microwell array and/or in the microwell are arranged in a monolayer.

In a further preferred embodiment of the present invention, the cell module, in particular the hydrogel or solid carrier, in particular the cell arrangement pattern, preferably the microwell array, comprises cells or cell clusters, in particular endocrine cells, in particular clusters of endocrine cells.

Preferably, the cell module, preferably the hydrogel or solid carrier, in particular the cell arrangement pattern, preferably the microwell array, comprises endocrine cells, differentiated cells derived from embryogenic or induced pluripotent stem cells (iPSC), allogeneic cells, autologous cells, xenogeneic cells or cells secreting drugs, antibodies and/or hormones.

Particularly preferred, the endocrine cells or endocrine cell clusters are pancreatic islets, pancreatic pseudo islets, stem cell-derived islet-like cell clusters, pancreatic beta-like cells, immature pancreatic beta cells, pancreatic beta cells or adrenocortical hormones-producing cells. Particularly preferred, the endocrine cells are pancreatic beta cells.

According to a preferred embodiment of the present invention, the endocrine cells are autologous cells. In another preferred embodiment, the endocrine cells are allogeneic cells. Preferably, the endocrine cells are xenogeneic cells. In a further preferred embodiment of the present invention, the endocrine cells are stem cell-derived endocrine cells.

In a further preferred embodiment of the present invention, the cell module, preferably the hydrogel or solid carrier, in particular the cell arrangement pattern, preferably the microwell array, comprises cell clusters having a diameter, in particular an average diameter, of at least 30 µm, preferably at least 40 µm, preferably at least 50 µm, preferably at least 60 µm, preferably at least 70 µm, preferably at least 80 µm, preferably at least 90 µm, preferably at least 100 µm, preferably at least 110 µm, preferably at least 120 µm, preferably at least 130 µm, preferably at least 140 µm, preferably at least 150 µm, preferably at least 160 µm, preferably at least 170 µm, preferably at least 180 µm, preferably at least 190 µm, preferably at least 200 µm.

Particularly preferred, the cell module, preferably the hydrogel or solid carrier, in particular the cell arrangement pattern, preferably the microwell array, comprises cell clusters having a diameter, in particular an average diameter, of at most 300 µm, preferably at most 290 µm, preferably at most 280 µm, preferably at most 270 µm, preferably at most 260 µm, preferably at most 250 µm, preferably at most 240 µm, preferably at most 230 µm, preferably at most 220 µm, preferably at most 210 µm, preferably at most 200 µm, preferably at most 190 µm, preferably at most 180 µm, preferably at most 170 µm, preferably at most 160 µm, preferably at most 150 µm, preferably at most 140 µm, preferably at most 130 µm, preferably at most 120 µm, preferably at most 110 µm, preferably at most 100 µm, preferably at most 90 µm, preferably at most 80 µm, preferably at most 70 µm, preferably at most 60 µm, preferably at most 50 µm, preferably at most 40 µm, preferably at most 30 µm.

According to a preferred embodiment of the present invention, the cell module, preferably the hydrogel or solid carrier, in particular the cell arrangement pattern, preferably the microwell array, comprises cell clusters having a diameter, in particular an average diameter, of 40 to 300 µm, preferably 50 to 250 µm, preferably 60 to 200 µm, preferably 70 to 150 µm, preferably 75 to 125 µm, preferably 80 to 120 µm, preferably 90 to 110 µm.

Particularly preferred, at least 60%, preferably at least 65%, preferably at least 70%, preferably at least 75%, preferably at least 80%, preferably at least 85%, preferably at least 90%, preferably at least 95%, preferably at least 96%, preferably at least 97%, preferably at least 98%, preferably at least 99%, of the cell clusters in the cell module, preferably in the hydrogel or solid carrier, in particular in the cell arrangement pattern, preferably in the microwell array, have a diameter of 40 to 300 µm, preferably 50 to 250 µm, preferably 60 to 200 µm, preferably 70 to 150 µm, preferably 75 to 125 µm, preferably 80 to 120 µm, preferably 90 to 110 µm.

In a preferred embodiment, the at least one oxygen module is configured to release oxygen in the shell. Particularly preferred, the at least one oxygen module comprises at least one oxygen-releasing material.

Preferably, the at least one oxygen-releasing material is a peroxide. Particularly preferred, the at least one oxygen-releasing material is a peroxide selected from the group consisting of calcium peroxide, magnesium peroxide, sodium peroxide, potassium peroxide, lithium peroxide, benzoylperoxide, hydrogen peroxide, and combinations thereof.

In a preferred embodiment, the at least one oxygen module comprises at least two different oxygen-releasing materials, in particular comprises at least two different peroxides. Preferably, the at least two different oxygen-releasing materials are selected from the group consisting of calcium peroxide, magnesium peroxide, sodium peroxide, potassium peroxide, lithium peroxide, benzoylperoxide, hydrogen peroxide, and combinations thereof.

According to a preferred embodiment of the present invention, the at least one oxygen-releasing material of the oxygen module is embedded in a carrier material.

In a preferred embodiment, the carrier material is a hydrophobic polymer, in particular a hydrophobic polymer selected from silicone, polyolefins, polyesters, polystyrene and co-polymers and mixture thereof. Preferably, the hydrophobic polymer is selected from poly(lactic-co-glycolic acid) (PLGA), polyvinylpyrrolidone (PVP), poly(methyl methacrylate) (PMMA), and silicone, preferably polydimethylsiloxane (PDMS). Preferably, the carrier material comprises, in particular consists of, silicone. In a particularly preferred embodiment, the silicone is a medical-grade silicone, preferably medical-grade polydimethylsiloxane (PDMS).

Preferably, the at least one oxygen module is arranged in the form of a layer comprising at least one oxygen-releasing material. In particular, the at least one oxygen module can be arranged in the form of a disk comprising at least one oxygen-releasing material.

According to this embodiment, the layer comprising at least one oxygen-releasing material, in particular the disk comprising at least one oxygen-releasing material, preferably has a thickness of 0.1 to 10 mm, preferably 0.25 to 8 mm, preferably 0.5 to 6 mm, preferably 0.75 to 4 mm, preferably 1 to 3 mm.

In a further preferred embodiment, the layer comprising at least one oxygen-releasing material, in particular the disk comprising at least one oxygen-releasing material, has a thickness of at least 0.1 mm, preferably at least 0.25 mm, preferably at least 0.5 mm, preferably at least 0.75 mm, preferably at least 1 mm.

Particularly preferred, the layer comprising at least one oxygen-releasing material, in particular the disk comprising at least one oxygen-releasing material, has a thickness of at most 10 mm, preferably at most 9 mm, preferably at most 8 mm, preferably at most 7 mm, preferably at most 6 mm, preferably at most 5 mm, preferably at most 4 mm, preferably at most 3 mm, preferably at most 2 mm, preferably at most 1 mm.

In another preferred embodiment of the present invention, the oxygen module is arranged in the form of particles comprising at least one oxygen-releasing material.

According to this embodiment, the particles comprising at least one oxygen-releasing material preferably have a particle size of 1 to 2000 µm, preferably 5 to 1750 µm, preferably 10 to 1500 µm, preferably 25 to 1250 µm, preferably 50 to 1000 µm, preferably 75 to 750 µm, preferably 100 to 500 µm.

In a further preferred embodiment, the particles comprising at least one oxygen-releasing material have a particle size of at least 1 µm, preferably at least 5 µm, preferably at least 10 µm, preferably at least 25 µm, preferably at least 50 µm, preferably at least 75 µm, preferably at least 100 µm, preferably at least 250 µm, preferably at least 500 µm.

Particularly preferred, the particles comprising at least one oxygen-releasing material have a particle size of at most 2000 µm, preferably at most 1750 µm, preferably at most 1500 µm, preferably at most 1250 µm, preferably at most 1000 µm, preferably at most 750 µm, preferably at most 500 µm, preferably at most 250 µm, preferably at most 100 µm.

In a preferred embodiment of the present invention, the particles comprising at least one oxygen-releasing material are homogeneously distributed in the cell module, in particular in the hydrogel or solid carrier. In a further preferred embodiment, the particles comprising at least oxygen-releasing material are inhomogeneously distributed in the cell module, in particular in the hydrogel or solid carrier.

Upon surgical implantation of the device encapsulating cells of interest, the surrounding environment is usually insufficiently vascularized to support the encapsulated cells with the required amount of oxygen. In this early phase after implantation, it is crucial for the viability of the encapsulated cells to ensure a constant supply with sufficient oxygen until the tissue surrounding the device is revascularized and the cells can be provided with oxygen by diffusion from capillaries of the host in close proximity of the device. The modular implantable device according to the present invention advantageously allows to bypass the initial lack of oxygen, in particular to supply cells encapsulated in the device with oxygen already during the early post transplantation phase, wherein the oxygen is released within the device by the at least one oxygen module. Preferably, molecular oxygen is generated within the device by the hydrolytic decomposition of the at least one oxygen-releasing material in the oxygen module.

In a preferred embodiment of the present invention, the immuno-isolating membrane is a composite membrane. Preferably, the immuno-isolating membrane is a membrane with selective permeability, preferably a composite membrane with selective permeability. Particularly preferred, the selective permeability of the immuno-isolating membrane, preferably of the composite membrane, is based on size exclusion and/or charge exclusion.

In a further preferred embodiment, the immuno-isolating membrane, in particular the composite membrane, comprises a meso- or macroporous membrane, preferably a mesoporous membrane, preferably a macroporous membrane, and a hydrogel, preferably a glycosaminoglycan (GAG)-based hydrogel or a synthetic highly sulfated or sulfonated hydrogel.

The meso- or macroporous membrane of the immuno-isolating membrane, in particular of the composite membrane, particularly serves to provide a mechanically strong basis for the immuno-isolating membrane, in particular for the composite membrane. The meso- or macroporous membrane particularly functions as a reinforcing element of the immuno-isolating membrane, in particular of the composite membrane.

The meso- or macroporous membrane, preferably has a pore size suitable to hinder passage of cells and macromolecules of the immune system through the membrane module of the modular implantable device.

In a particularly preferred embodiment of the present invention, the meso- or macroporous membrane of the immuno-isolating membrane, in particular of the composite membrane, has a pore size, preferably an average pore size, of 25 to 1000 nm, preferably 50 to 900 nm, preferably 75 to 800 nm, preferably 100 to 700 nm, preferably 200 to 600 nm, preferably 300 to 500 nm, preferably 350 to 450 nm.

Preferably, the meso- or macroporous membrane of the immuno-isolating membrane, in particular of the composite membrane, has a pore size, preferably an average pore size, of at most 1000 nm, preferably at most 900 nm, preferably at most 800 nm, preferably at most 700 nm, preferably at most 650 nm, preferably at most 600 nm, preferably at most 550 nm, preferably at most 500 nm.

In another preferred embodiment, the meso- or macroporous membrane of the immuno-isolating membrane, in particular of the composite membrane, has a pore size, preferably an average pore size, of at least 25 nm, preferably at least 50 nm, preferably at least 75 nm, preferably at least 100 nm, preferably at least 150 nm, preferably at least 200 nm, preferably at least 250 nm, preferably at least 300 nm, preferably at least 350 nm, preferably at least 400 nm.

In a preferred embodiment of the present invention, the meso- or macroporous membrane, preferably the mesoporous membrane, preferably the macroporous membrane, comprises expanded polytetrafluoroethylene (ePTFE), in particular consists of ePTFE.

Particularly preferred, the ePTFE in the immuno-isolating membrane is hydrophilized.

The hydrogel of the immuno-isolating membrane, in particular the GAG-based hydrogel or the synthetic highly sulfated or sulfonated hydrogel of the immuno-isolating membrane, preferably of the composite membrane, forms a network and preferably prohibits the passage of cells and macromolecules, in particular cells and macromolecules of the immune system.

In a preferred embodiment, the hydrogel of the immuno-isolating membrane, in particular the GAG-based hydrogel or the synthetic highly sulfated or sulfonated hydrogel of the immuno-isolating membrane, preferably of the composite membrane, hinders the passage of molecules with a hydrodynamic diameter of 12 nm or more, preferably 11 nm or more, preferably 10 nm or more, preferably 9 nm or more, preferably 8 nm or more, preferably 7 nm or more, preferably 6 nm or more, preferably 5 nm or more, preferably 4 nm or more.

According to a preferred embodiment of the present invention, the hydrogel of the immuno-isolating membrane, in particular the GAG-based hydrogel or the synthetic highly sulfated or sulfonated hydrogel of the immuno-isolating membrane, preferably of the composite membrane, has a mesh size, preferably an average mesh size, of 4 to 16 nm, preferably 5 to 15 nm, preferably 6 to 14 nm, preferably 7 to 13 nm, preferably 8 to 12 nm, preferably 9 to 11 nm.

In a further preferred embodiment, the hydrogel of the immuno-isolating membrane, in particular the GAG-based hydrogel or the synthetic highly sulfated or sulfonated hydrogel of the immuno-isolating membrane, preferably of the composite membrane, has a mesh size, preferably an average mesh size, of at most 20 nm, preferably at most 19 nm, preferably at most 18 nm, preferably at most 17 nm, preferably at most 16 nm, preferably at most 15 nm, preferably at most 14 nm, preferably at most 13 nm, preferably at most 12 nm, preferably at most 11 nm, preferably at most 10 nm, preferably, at most 9 nm, preferably at most 8 nm, preferably at most 7 nm, preferably at most 6 nm.

Preferably, the hydrogel of the immuno-isolating membrane, in particular the GAG-based hydrogel or the synthetic highly sulfated or sulfonated hydrogel of the immuno-isolating membrane, preferably of the composite membrane, has a mesh size, preferably an average mesh size, of at least 4 nm, preferably at least 4.5 nm, preferably at least 5 nm, preferably at least 6 nm, preferably at least 7 nm, preferably at least 8 nm, preferably at least 9 nm, preferably at least 10 nm, preferably at least 11 nm, preferably at least 12 nm.

Accordingly, the mesh size of the hydrogel of the immuno-isolating membrane, in particular the GAG-based hydrogel or the synthetic highly sulfated or sulfonated hydrogel of the immuno-isolating membrane, preferably of the composite membrane, allows passage of molecules required for the metabolism of cells and secreted by cells encapsulated in the modular implantable device, such as oxygen, glucose, hormones and salts, but excludes cells and certain molecules of the immune system from passage through the at least one immuno-isolating membrane of the modular implantable device.

In a preferred embodiment of the present invention, the hydrogel of the immuno-isolating membrane, in particular the GAG-based hydrogel or the synthetic highly sulfated or sulfonated hydrogel of the immuno-isolating membrane, preferably of the composite membrane, is not degradable.

Preferably, the hydrogel is a synthetic hydrogel, preferably a synthetic highly sulfated or sulfonated hydrogel, or a semi-synthetic GAG-based hydrogel, preferably a multi-armed polyethylene glycol (starPEG) heparin hydrogel. Particularly preferred, the starPEG heparin hydrogel comprises, in particular consists of, heparin and starPEG. In a preferred embodiment, the starPEG heparin hydrogel is formed by covalent crosslinking of heparin and starPEG. Preferably, the starPEG heparin hydrogel is formed by covalent crosslinking of heparin and starPEG using ethyl(dimethylaminopropyl) carbodiimide (EDC) and sulfo-N-hydroxysuccinimide (sNHS).

In a preferred embodiment of the present invention, the immuno-isolating membrane, in particular the composite membrane, comprises a meso- or macroporous membrane, preferably a meso- or macroporous membrane comprising ePTFE, which is impregnated with a hydrogel, in particular a GAG-based hydrogel or a synthetic highly sulfated or sulfonated hydrogel, preferably a starPEG heparin hydrogel.

Preferably, the hydrogel of the at least one immuno-isolating membrane, in particular of the at least one composite membrane, is suitable to scavenge cytokines, in particular pro-inflammatory cytokines, preferably MCP-1 (monocyte chemoattractant protein-1), IL-8 (interleukin-8), MIP-1α (macrophage inflammatory protein-la) and MIP-1β (macrophage inflammatory protein-1β). In a particularly preferred embodiment of the present invention, the hydrogel of the at least one immuno-isolating membrane, in particular of the at least one composite membrane, is a starPEG heparin hydrogel.

According to a preferred embodiment of the present invention, the at least one immuno-isolating membrane, in particular the at least one composite membrane, is further coated with at least one hydrogel, in particular with at least one layer of hydrogel. Particularly preferred, the at least one hydrogel is a degradable hydrogel, in particular a biodegradable hydrogel. In this particular embodiment, the at least one hydrogel layer serves to further increase the integration of the modular implantable device into the body.

The present invention also encompasses embodiments in which the immuno-isolating membrane, in particular the composite membrane, further comprises at least one bioactive compound. Preferably, the at least one bioactive compound is at least one bioactive molecule, preferably at least one growth factor, in particular at least one pro-angiogenic/vascularising growth factor and/or at least one trophic growth factor.

Preferably, the at least one bioactive compound, in particular the at least one growth factor, is selected from the group consisting of vascular endothelial growth factors (VEGF)s, fibroblast growth factor (FGF), tumor necrosis factor-alpha (TNF-α), transforming growth factor-alpha (TGF-α), transforming growth factor-beta (TGF-β), Ephrins, epidermal growth factor (EGF), growth hormone, Follistatin-like protein 1 (FSTL1), hepatocyte growth factor (HGF), insulin-like growth factors (IGF)s, interleukins (IL)s, platelet-derived growth factor (PDGF), leptin, thrombopoietin (TPO), trefoil factor peptides (TFF), sonic hedgehog (Shh), angiopoietins (Ang) and stromal cell-derived factor 1 (SDF-1).

In another preferred embodiment of the present invention, the at least one oxygen module is arranged, in particular embedded or enclosed, between two brackets configured for accommodating at least one cell module, wherein the at least one oxygen module is contained in a shell formed by the brackets and at least one immuno-isolating membrane.

According to a further preferred embodiment of the present invention, the at least one bracket comprises, in particular consists of, at least one material selected from polyether ether ketone (PEEK), silicone, preferably PDMS, PTFE, polyethylene (PE), polypropylene (PP), polymethacrylates, polyesters, polyamides, and polyurethanes.

Preferably, the at least one bracket comprises, in particular consists of, PEEK, silicone, preferably PDMS, and/or PTFE. Particularly preferred, the at least one bracket comprises, in particular consists of, PEEK. In another preferred embodiment of the present invention, at least one bracket comprises, in particular consists of, silicone, preferably PDMS. Preferably, the at least one bracket comprises, in particular consists of, PTFE.

In a particularly preferred embodiment of the present invention, the modular implantable device has a planar geometry. Preferably, the modular implantable device has a polygonal or round geometry, in particular a polygonal or round planar geometry, preferably a round planar geometry.

According to a preferred embodiment of the present invention, the modular implantable device is a bioartificial organ comprising endocrine cells or endocrine cell clusters. Preferably, the modular implantable device is a bioartificial organ comprising endocrine cells or endocrine cell clusters suitable for use in the treatment of endocrine function-related diseases and disorders in human or animal subjects.

Preferably, the endocrine cells or endocrine cell clusters are pancreatic islets, pancreatic pseudo-islets, stem cell-derived islet-like cell clusters, pancreatic beta-like cells, immature pancreatic beta cells, pancreatic beta cells or adrenocortical hormones producing cells. Particularly preferred, the endocrine cells are pancreatic beta cells.

In a preferred embodiment of the present invention, the modular implantable device is a bioartificial organ comprising pancreatic islets, pancreatic pseudo islets, stem cell-derived islet-like cell clusters, pancreatic beta-like cells, immature pancreatic beta cells and/or pancreatic beta cells suitable for use in the treatment of blood glucose-related diseases and disorders in human or animal subjects, in particular suitable for use in the treatment of diabetes mellitus (DM) in human or animal subjects, preferably suitable for use in the treatment of diabetes mellitus type 1 in human or animal subjects.

The present invention further pertains to a method of preparing a modular implantable device, in particular a modular implantable device according to the present invention, comprising:
a) providing at least one bracket configured for accommodating at least one cell module within the device, at least one oxygen module, at least one cell module and at least one immuno-isolating membrane,
b) loading the cell module, in particular a hydrogel or solid carrier, preferably a cell arrangement pattern, with cells, preferably isolated endocrine cells,
c) assembling the modular implantable device by arranging that at least one oxygen module and the at least one cell module within the device in a shell formed by the at least one bracket and the at least one immuno-isolating membrane.

In a particularly preferred embodiment of the present invention, the cells, preferably isolated endocrine cells, loaded into the cell module, in particular the hydrogel or solid carrier, preferably the cell arrangement pattern, in step b), are allowed to settle in the cell module, in particular in the hydrogel or solid carrier, preferably in the cell arrangement pattern, before the modular implantable device is assembled in step c).

According to a further preferred embodiment of the present invention, the cells, preferably isolated endocrine cells, loaded into the cell module, in particular the hydrogel or solid carrier, preferably the cell arrangement pattern, in step b), are allowed to form cell clusters before the modular implantable device is assembled in step c).

In a preferred embodiment of the present invention, two cell modules are provided in step a), wherein the at least one oxygen module is arranged, in particular embedded or enclosed, between the two cell modules, and wherein the two cell modules and the at least one oxygen module are contained within the device in a shell formed by the at least one bracket and the at least one immuno-isolating membrane in step c).

In a further aspect, the present invention pertains to the use of a modular implantable device according to the present invention for the treatment of the endocrine function-related diseases and disorders. In a particular preferred embodiment of the present invention, the endocrine function-related diseases and disorders are selected from diabetes mellitus type 1 and adrenal insufficiency, in particular adrenocortical insufficiency.

The present invention also pertains to the use of a modular implantable device according to the present invention for the treatment of blood glucose-related diseases and disorders. In a preferred embodiment of the present invention, the blood glucose-related disease and disorder is diabetes mellitus (DM), preferably diabetes mellitus type 1.

Another aspect of the present invention is directed to a method of implanting a modular implantable device according to the present invention into the human or animal body, comprising the steps:
a) providing at least one bracket configured for accommodating at least one cell module within the device, at least one oxygen module, at least one cell module and at least one immuno-isolating membrane,
b) loading the cell module, in particular a hydrogel or solid carrier, preferably a cell arrangement pattern, with cells, preferably isolated endocrine cells,
c) assembling the modular implantable device by arranging that at least one oxygen module and the at least one cell module within the device in a shell formed by the at least one bracket and the at least one immuno-isolating membrane.
d) implanting the modular implantable device into the human or animal body.

All information and embodiments provided in connection with the modular implantable device according to the present invention apply *mutatis mutandis* also to the method of preparing a modular implantable device, the uses of a modular implantable device according to the present invention, and the method of implanting a modular implantable device according to the present invention.

In the context of the present invention, the term "macroencapsulation" pertains to the encapsulation of a plurality of cell or cell clusters, in particular of a functionally interacting group of cells, such as tissues, in a shielded space.

The term "solid carrier" is used herein to designate a solid material suitable for accommodating a second material, in particular suitable for arranging a second material in a particular position.

The expression "hydrogel" designates a gel comprising water-insoluble polymers with a high water absorbing capacity which are covalently or non-covalently interconnected to form a three-dimensional network. The polymers forming a hydrogel can be of natural or artificial origin.

The term "impregnated" is used in the context of the present invention to describe the condition of a first material, in which at least the porous structure of the material is saturated with a second material. In the context of the present invention, the expression "an expanded polytetrafluoroethylene (ePTFE) material which is impregnated with a hydrogel" is used to designate that the porous structure of the ePTFE material is saturated with a hydrogel material, in particular that the hydrogel material infiltrated the pores of the ePTFE material.

In the context of the present invention, a "bracket" is a structure which is configured to fix or support, in particular to hold and/or receive, an object. Accordingly, the expression "at least one bracket configured for accommodating at least one cell module within the device" refers to at least one structure which is formed in a way to allow for holding and/or receiving at least one cell module. A "bracket" can either be an isolated separate structure or part of another structure. In a particular embodiment of the present invention, the "at least one bracket" can be part of a "membrane module" and/or of a "scaffold module". In other embodiments, the "at least one bracket" can be a separate structure within the modular implantable device of the present invention.

A "scaffold module" as used herein refers to an optional sub-compartment of the modular implantable device according to the present invention which provides the device with a certain mechanical strength. In a preferred embodiment, the "scaffold module" can harbour at least one bracket for accommodating at least one cell module within the device. The "scaffold module" can be formed of various suitable materials providing the desired mechanical strength to an implantable device.

The term "membrane module" is used in the context of the present invention for an optional sub-compartment of the modular implantable device, which comprises at least one immuno-isolating membrane. The "membrane module" can further harbour at least one bracket for accommodating at least one cell module within the device. In a preferred embodiment, the "membrane module" comprises means to support the at least one immuno-isolating membrane.

In the context of the present invention, the term "immuno-isolating membrane" is used for a perm-selective membrane, in particular a size-selective membrane, i.e. a semi-permeable membrane which is based on the principle of size exclusion. Such membrane is configured to hinder the passage of components exceeding a particular hydrodynamic diameter and allows for the passage of components having a hydrodynamic diameter below the particular hydrodynamic diameter. In addition thereto, the selective permeability of the immuno-isolating membrane can further be based on the principle of charge exclusion. Particularly preferred, the negative charge of the hydrogel, preferably the synthetic highly sulfated or sulfonated hydrogel or the GAG-based hydrogel, in particular the starPEG heparin hydrogel, of the "immuno-isolating membrane" electrostatically binds positively charged biomolecules. According to the present invention, the "immuno-isolating membrane" in particular allows for the passage of molecules required by or secreted by the cells encapsulated in the modular implantable device according to the present invention, such as oxygen, glucose, hormones and salts, but excludes cells and certain molecules of the immune system from passage therethrough. Accordingly, the diffusion of components, in particular molecules, through the "immuno-isolating membrane" is dependent on the hydrodynamic diameter and the charge of the components, in particular on the hydrodynamic diameter and the charge of the molecules. Besides its function of size and charge exclusion, the hydrogel of the "immuno-isolating membrane" can further be configured to selectively bind particular bioactive molecules, such as proinflammatory cytokines, thereby regulating the immune response of the tissue surrounding the implanted device. The "immuno-isolating membrane" of the present invention can make up a part or the entirety of the surface of the modular implantable device. Accordingly, the modular implantable device of the present invention can comprise only one, two or a number of immuno-isolating membranes.

The term "mesoporous membrane" is used herein to describe a membrane comprising pores with a pore diameter, in particular an average pore diameter, of 2 nm und 50 nm. In the context of the present invention, the term "macroporous membrane" is used for a membrane comprising pores with a pore diameter, in particular an average pore diameter, of at least 50 nm.

The expression "oxygen module" is used in the context of the present invention to designate a sub-compartment of the modular implantable device according to the present invention which releases oxygen within the immunologically shielded space of the device. The "oxygen module" can be of any conceivable form, in particular in the form of a layer or in the form of particles, and can comprise at least one oxygen-releasing material, in particular at least one peroxide. The "oxygen module" according to the present invention particularly serves to supply molecular oxygen to cells encapsulated within the device, in particular in the early post-translational phase.

In the context of the present invention, the expression "cell arrangement pattern" refers to a particular arrangement of cells or cell clusters with respect to one another and/or with respect to the components of the modular implantable device. Thus, the "cell arrangement pattern" serves to position the cells or cell clusters at particular locations within the cell module device. According to the present invention, the "cell arrangement pattern" can either be a regular or an irregular arrangement of cells or cell clusters with respect to one another and/or with respect to the components of the modular implantable device. Particularly preferred, the "cell arrangement pattern" of the cell module is a regular repetitive arrangement of cells or cells clusters. In a preferred embodiment, the "cell arrangement pattern" can be an array of cells or cell clusters, such as an arrangement of the cells or cell clusters in the form of a row (one-dimensional array), a grid (two-dimensional array), or a stacked array (three-dimensional array). The way in which the "cell arrangement pattern" is structurally formed is not restricted. Preferably, cells or cells clusters are arranged in or on a hydrogel or solid carrier with respect to one another to create the "cell arrangement pattern", in particular a regular repetitive "cell arrangement pattern". In this regard, the cells or cell clusters can for example be embedded in a hydrogel. The present invention also encompasses embodiments in which the cells or cell clusters are positioned in indentations or on or inside elevations of a hydrogel or solid carrier.

The expression "surface of the cells or the surface of the cell clusters" as used herein is directed to the surface of the cells or cells clusters which is closest to the respective reference point, such as the "immuno-isolating membrane" or the "oxygen module".

In the context of the present invention, the term "a" is meant to include the meaning of "one" or "one or more". The term "comprising" preferably has the meaning of "containing" or "including", meaning including the specifically identified elements without excluding the presence of further elements. However, in a preferred embodiment, the term "comprising" is also understood to have the meaning of "consisting essentially of' and in a further preferred embodiment the meaning of "consisting of'.

The expression "and/or" is used herein to express the disclosure of any combination of individual elements connected within a listing. Solely for illustrative purpose, the expression "A, C, and/or C" can mean A individually, B individually, C individually, (A and B), (B and C), (A and C), and (A, B and C).

Further preferred embodiments of the present invention are subject of the subclaims. The invention is described in the following by way of an example and the accompanying figures.

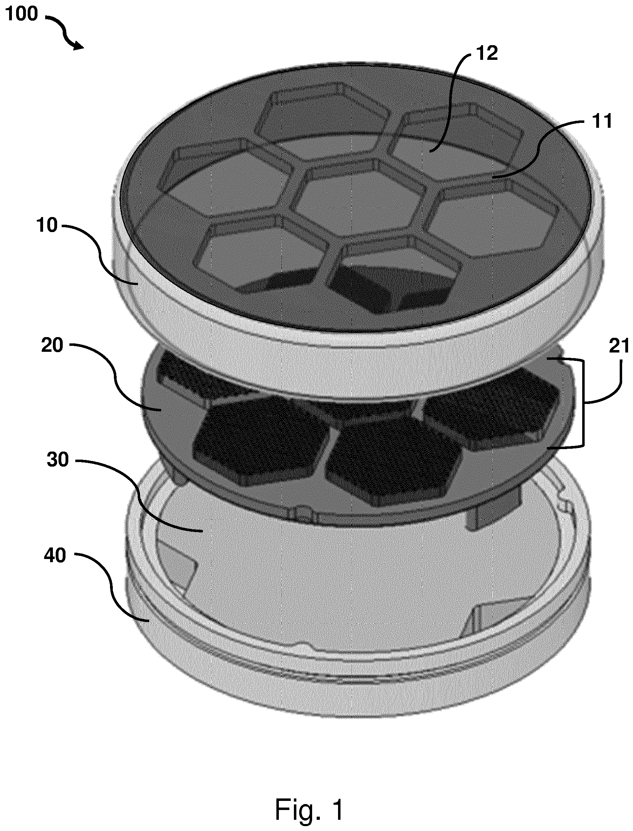

**Figure 1** shows a modular implantable device (100) comprising a scaffold module (40), an oxygen module (30), a cell module (20) comprising a microwell array (21), and a membrane module (10) comprising a bracket (11) and an immuno-isolating membrane (12).

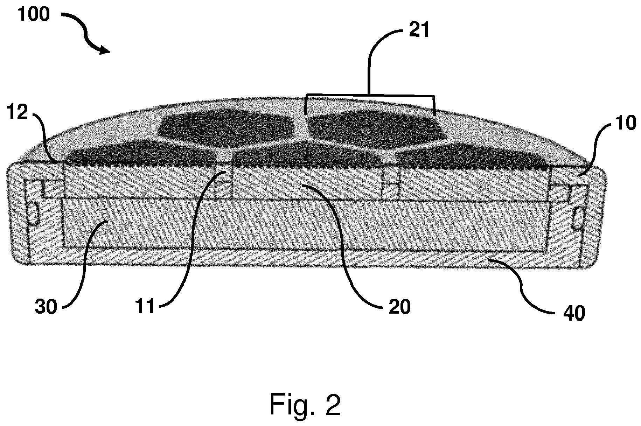

In **Figure 2****,** a cross sectional view of a modular implantable device (100) is shown. The modular implantable device (100) comprises a scaffold module (40) in which an oxygen module (30) is positioned below a cell module (20) comprising a microwell array (21). A membrane module (10) comprising a bracket (11) and an immuno-isolating membrane (12) accommodates the cell module within the device (100).

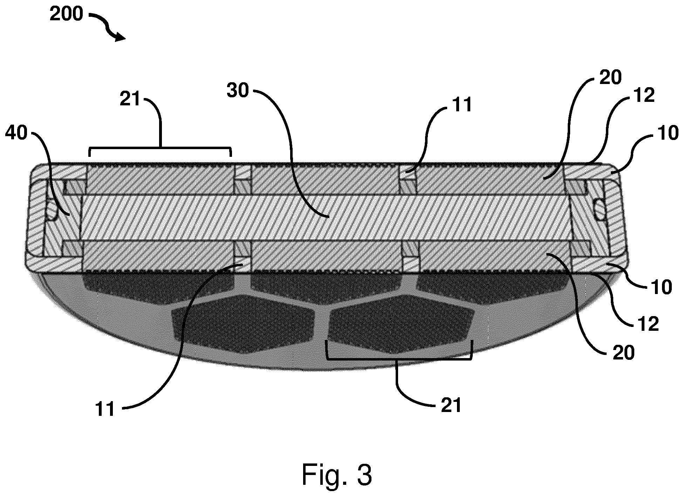

**Figure 3** illustrates an embodiment of a modular implantable device (200) which comprises a scaffold module (40), an oxygen module (30) and two cell modules (20) each comprising one or more microwell array (21), wherein the oxygen module (30) and the two cell modules (20) are contained in a shell formed by two brackets (11) of membrane modules (10) and two immuno-isolating membranes (12).

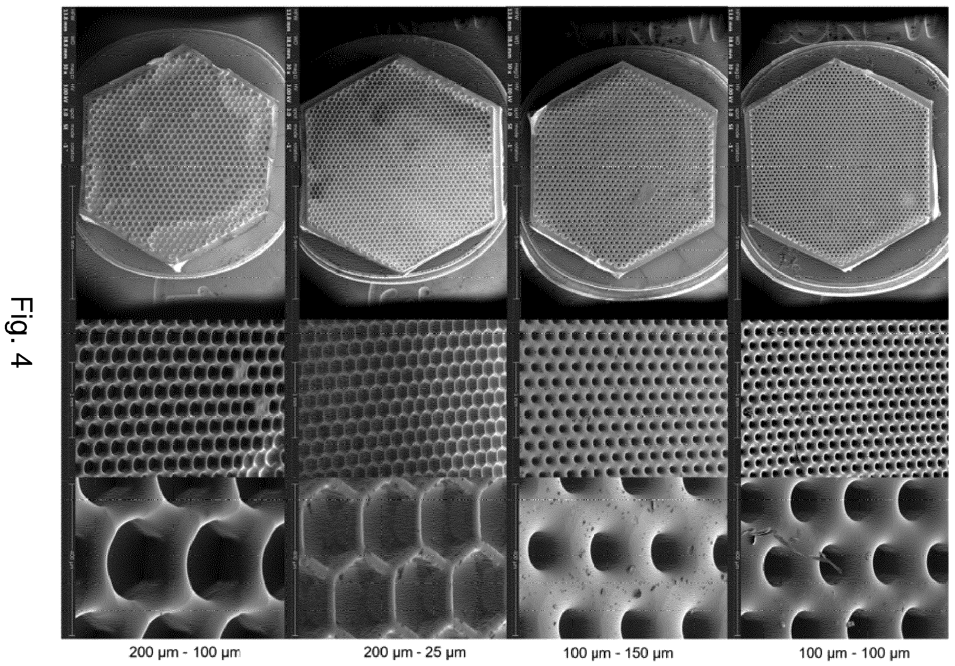

**Figure 4** shows electron microscopic images of microwell hydrogel arrays with different geometries of the microwells and different spacing between the individual wells.

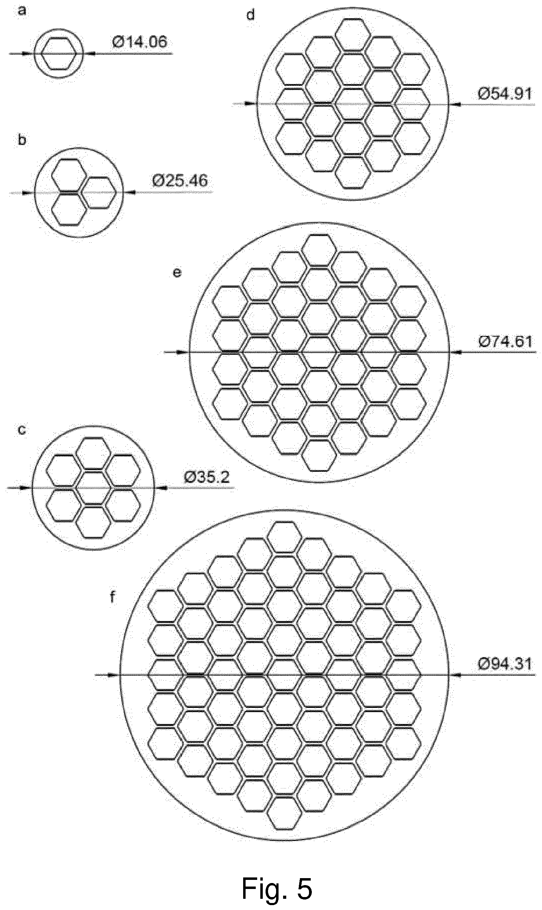

**Figure 5** shows cell modules comprise one **(a),** three **(b),** seven **(c),** 19 **(d),** 37 **(e),** and 61 **(f)** repetitive hexagonal units (microwell hydrogel arrays, see **Figure 4**) which are arranged with respect to each other to form a triangular (see **Figure 5** **b**) or hexagonal superstructure (see Figure 5 c-f).

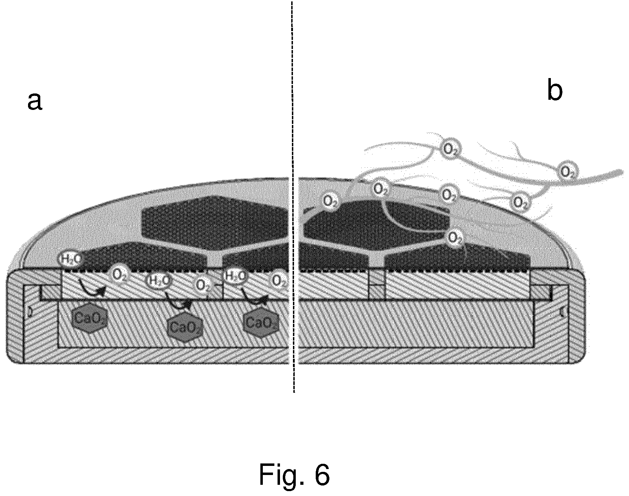

In **Figure 6** a comparison of the oxygen supply to cells in the cell module of a modular implantable device during the early post-transplantation phase **(a)** and after revascularisation **(b)** is shown. In the early post-transplantation phase **(a),** oxygen is primarily supplied to the cells or cell clusters in the cell module by chemical decomposition of an oxygen-releasing material (e.g. calcium peroxide (CaO₂)) in the oxygen module. After the tissue surrounding the implant has been sufficiently revascularized **(b),** the cells or cell clusters in the cell module are primarily provided with oxygen by free diffusion from capillaries in close proximity to the device.

### Example:

### Loading and assembly of a modular implantable device

1. A funnel is placed onto the cell module containing the cell arrangement patterns.
2. A suspension of single endocrine cells is pipetted onto the cell module.
3. Cells are allowed to settle and cluster inside the indentations of the cell arrangement pattern.
4. The cell module is combined with the oxygen module and immune-isolating membrane into the bracket.
5. A hydrogel coating for improved ingrowth of the implant is applied onto the surface of the implant.
6. Transplantation of the device.

## Claims

1. A modular implantable device for the macroencapsulation of cells, comprising:
- at least one bracket configured for accommodating at least one cell module within the device,
- at least one oxygen module, and
- at least one immuno-isolating membrane,
wherein the at least one oxygen module is contained within the device in a shell formed by the at least one bracket and the at least one immuno-isolating membrane, optionally together with the at least one cell module.

2. The modular implantable device according to claim 1, wherein a cell module is arranged in the at least one bracket configured for accommodating at least one cell module in the device.

3. The modular implantable device according to claim 1 or 2, wherein the cell module comprises a hydrogel or a solid carrier comprising a cell arrangement pattern, in particular a microwell array, preferably a microwell hydrogel array.

4. The modular implantable device according to claim 3, wherein the hydrogel or solid carrier further comprises at least one bioactive compound, preferably at least one bioactive compound effecting survival, functionality and/or maturation of cells.

5. The modular implantable device according to any one of the preceding claims, wherein the at least one oxygen module is configured to release oxygen in the shell, in particular comprises an oxygen-releasing material.

6. The modular implantable device according to any one of the preceding claims, wherein the hydrogel is a natural, semi-synthetic or synthetic polymer, in particular selected from the group consisting of glycosaminoglycans (GAGs), agarose, chitosan, alginate, collagen, poly(amino acid)s, poly(peptoid)s and derivatives thereof, poly(ethylene glycol)s (PEG), poly(oxazoline)s (POX), synthetic sulfonated or sulfated polymers, e.g. sulfonated or sulfated poly(acrylic acid) or poly(styrene sulfonic acid), acrylates, poly(N-vinyl-2-pyrrolidinone) (PNVP), poly(acrylamide) (PAM), poly(vinyl alcohol) (PVA), poly(ethylene oxide) (PEO), poly(N-isopropyl acrylamide) (PNIPAM), poly(methacrylic acid) (PMAA), and derivatives thereof, and combinations thereof.

7. The modular implantable device according to any one claims 3 to 6, wherein the cell arrangement pattern, in particular the microwell array and/or the microwell, has a hexagonal geometry.

8. The modular implantable device according to any one claims 5 to 7, wherein the oxygen-releasing material is a peroxide, in particular a peroxide selected from the group consisting of calcium peroxide, magnesium peroxide, sodium peroxide, potassium peroxide, lithium peroxide, benzoylperoxide, hydrogen peroxide, and combinations thereof.

9. The modular implantable device according to any one claims 5 to 8, wherein the oxygen-releasing material of the oxygen module is embedded in a carrier material, preferably a hydrophobic polymer, in particular silicone, preferably polydimethylsiloxane (PDMS).

10. The modular implantable device according to any one of the preceding claims, wherein the immuno-isolating membrane is a composite membrane comprising a meso- or macroporous membrane, preferably expanded polytetrafluoroethylene (ePTFE), and a GAG-based hydrogel or a synthetic highly sulfated or sulfonated hydrogel.

11. The modular implantable device according to claim 10, wherein the GAG-based hydrogel is a semi-synthetic hydrogel, preferably a starPEG heparin hydrogel.

12. The modular implantable device according to any one of the preceding claims, wherein the at least one bracket comprises polyether ether ketone (PEEK), silicone, and/or PTFE.

13. The modular implantable device according to any one of the preceding claims, wherein the wells of the microwell array have a width of at most 350 µm, preferably at most 300 µm, preferably at most 200 µm, preferably at most 150 µm, preferably at most 125 µm, preferably at most 100 µm, and/or are spaced from each other by at least 5 µm, preferably at least 10 µm, preferably at least 25 µm.

14. The modular implantable device according to any one of the preceding claims, wherein the oxygen module is arranged between two brackets configured for accommodating at least one cell module, and wherein the oxygen module is contained in a shell formed by the brackets and at least one immuno-isolating membrane.

15. The modular implantable device according to any one of the preceding claims, wherein the cell module, preferably the hydrogel or solid carrier, in particular the cell arrangement pattern, in particular the microwell array, comprises cells or cell clusters, in particular endocrine cells or endocrine cell clusters, preferably pancreatic islets, pancreatic pseudo islets, stem cell-derived islet-like cell clusters, pancreatic beta-like cells, immature pancreatic beta cells, pancreatic beta cells or adrenocortical hormones-producing cells, differentiated cells derived from embryogenic or induced pluripotent stem cells (iPSC), allogeneic cells, autologous cells, xenogeneic cells or cells secreting drugs, antibodies and/or hormones..

16. A method of preparing a modular implantable device, in particular a modular implantable device according to any of the preceding claims, comprising:
a) providing at least one bracket configured for accommodating at least one cell module within the device, at least one oxygen module, at least one cell module and at least one immuno-isolating membrane,
b) loading the cell module, in particular a hydrogel or a solid carrier, preferably a cell arrangement pattern, with cells, preferably isolated endocrine cells,
c) assembling the modular implantable device by arranging the at least one oxygen module and the at least one cell module within the device in a shell formed by the at least one bracket and the at least one immuno-isolating membrane.
